# EUROPÄISCHE PATENTSCHRIFT

(11) **EP 2 714 126 B1**
(45) Veröffentlichungstag und Bekanntmachung des Hinweises auf die Patenterteilung: **18.07.2018**
(21) Anmeldenummer: 12724292.3
(22) Anmeldetag: 22.05.2012
(51) Int. Cl.: A61M 1/16, A61M 1/36

(54) **VERFAHREN ZUM SPÜLEN UND/ODER ZUM BEFÜLLEN EINER BLUTBEHANDLUNGSVORRICHTUNG SOWIE BLUTBEHANDLUNGSVORRICHTUNG**
METHOD FOR FLUSHING AND/OR FILLING A BLOOD TREATMENT DEVICE, AND BLOOD TREATMENT DEVICE
PROCÉDÉ DE RINÇAGE ET/OU DE REMPLISSAGE D'UN DISPOSITIF DE TRAITEMENT DU SANG ET DISPOSITIF DE TRAITEMENT DU SANG

(30) Priorität: 24.05.2011 DE 102011102492; 24.05.2011 US 201161457740 P
(43) Veröffentlichungstag der Anmeldung: 09.04.2014
(60) Teilanmeldung: 18172705.8
(73) Patentinhaber: Fresenius Medical Care Deutschland GmbH, 61352 Bad Homburg (DE)
(72) Erfinder: GRONAU, Sören, 64569 Nauheim (DE); NOACK, Joachim, 97616 Bad Neustadt (DE); HAECKER, Jürgen, 61267 Neu-Anspach (DE); MÜLLER, Ralf, 61348 Bad Homburg (DE)
(74) Vertreter: Laufhütte, Dieter
(86) Internationale Anmeldenummer: PCT/EP2012/002174
(87) Internationale Veröffentlichungsnummer: WO 2012/159740

(56) Entgegenhaltungen:
- EP-A1- 1 892 000
- US-A1- 2005 131 332
- US-A1- 2009 076 433

## Beschreibung

Die vorliegende Erfindung betrifft ein Verfahren zum Spülen und/oder zum Befüllen einer Blutbehandlungsvorrichtung sowie eine Blutbehandlungsvorrichtung.

Vor der Inbetriebnahme von Blutbehandlungsvorrichtungen, insbesondere Dialysemaschinen, ist es erforderlich, beispielsweise vor einer Dialysebehandlung die Maschine entsprechend aufzurüsten und sowohl den Dialysekreislauf, den extrakorporalen Blutkreislauf als auch den eingesetzten Membranfilter bzw. Dialysefilter zu entlüften. Hierzu wird in der Regel der Dialysekreislauf, der extrakorporale Blutkreislauf als auch der den Dialysekreislauf vom Blutkreislauf semipermeabel trennende Dialysefilter gespült.

Um die Effizienz der Reinigung des Blutes zu erhöhen, strömt während der Behandlung das Blut in den Hohlfasern des Dialysators in entgegengesetzter Richtung zur Flußrichtung der Dialyselösung. Die Dialyselösung befindet sich dabei außerhalb der Hohlfasern des Dialysators. Da die Luft auf der Blutseite während der Behandlung als problematischer, insbesondere wegen etwaiger Luftinfusionen, die zu Luftembolien führen können, erachtet wird, als auf der Dialysatseite, werden die Dialysatoren derart angeordnet, daß das Blut während der Behandlung von unten nach oben durch den Dialysator strömt, während die Dialyselösung von oben nach unten strömt. Aus diesem Grund ist die dialysatseitige Luftentfernung, insbesondere bei großen Dialysatoren, häufig nicht optimal und es bleiben regelmäßig, auch für den Benutzer erkennbar, Restluftansammlungen im Dialysator, insbesondere auf der Dialysatseite zurück.

Diesem Umstand wird dadurch begegnet, daß durch das Bedienpersonal der Dialysestation der Dialysator gedreht wird und durch anschließendes oder gleichzeitiges Klopfen auf den Dialysator versucht wird, die restliche Luft aus dem Dialysator zu entfernen. Durch das Drehen wird der Dialysatausgang nach oben gelegt, wodurch Restluft besser aus dem Dialysator gespült werden kann. Hierdurch kann eine gute Entlüftung auf der Dialysatseite, insbesondere auf der Dialysatseite des Dialysators erreicht werden.

Als nachteilig erweist sich dabei, daß dieses Drehen manuell und zeitraubend durch den Benutzer durchzuführen ist, der Benutzer beim Klopfen den Dialysator möglicherweise beschädigen kann und auch erhöhte Kosten dadurch entstehen, daß die Dialysatorkonnektionsschläuche des Schlauchssystems sehr lang ausgeführt werden müssen, um ein Drehen des Dialysators zu ermöglichen. Hierdurch wird zugleich in nachteiliger Weise das extrakorporale Blutvolumen erhöht. Darüber hinaus wird die Entlüftung der Blutseite bei gedrehtem Filter verschlechtert, was insbesondere dann der Fall ist, wenn versehentlich der Dialysator nicht in die Ausgangsposition zurückgedreht wird, d. h., daß der Bluteinlaß wieder an dem unteren Ende des Dialysators befindlich ist und der Dialysateinlaß am oberen Ende des Dialysators befindlich ist.

Aus dem Stand der Technik sind bereits Vorrichtungen bekannt, mittels dessen das Drehen des Dialysators erleichtert werden kann.

So zeigt die DE 696 25 279 T2 eine Dialysemaschine, die einen Dialysatorhalter aufweist, mittels dessen der Dialysator während des Befüllens automatisch gedreht werden kann.

Alternativ sind bereits Verfahren bekannt, mittels derer der Schritt des Drehens des Dialysators vermieden werden soll.

So zeigt die DE 696 23 563 T3 ein Verfahren und eine Vorrichtung zur Spülung eines Dialysators, bei dem nach dem Füllen des extrakorporalen Blutkreislaufs im Spülkreislauf Druckwellen erzeugt werden, so daß anhaftende Luftblasen gelöst werden sollen. Zugleich wird auch eine Schaltung beschrieben, bei der im Spülmodus Blut- und Dialysatkompartiment von unten nach oben durchströmt werden.

In der WO 2004/43520 A1 wird ein Verfahren beschrieben, in dem zunächst die Fasern des Filters mittels der Blutpumpe von innen bei einem geringen Druck bzw. Fluß gefüllt und dann mit einem höheren Druck gespült werden. Sodann wird das Dialysat z. B. durch die Membran in den Dialysatraum gedrückt. Dort wird es sodann von der Dialysatpumpe zum Spülen umgepumpt, wobei zum Teil auch eine Richtungsumkehr möglich ist. Durch das Spülen bei höheren Flüssen soll die Luft entfernt werden. Gemäß der WO 2004/43520 A1 erfolgt die Füllung des Hämodialysators zunächst auf der Blutseite mit der Blutpumpe. Die Priminglösung wird von der Blutpumpe ins Innere der Hohlfasern gefördert und von dort durch die Membran auf die Dialysatseite gedrückt.

Aus der US 2005/0131332 A1 ist ein Verfahren zum Befüllen und Spülen eines Blutschlauchsatzes mit zwei in Serie geschalteten Dialysefiltern bekannt, für welches die Enden der venösen und der arteriellen Leitung des extrakorporalen Blutkreislaufs miteinander verbunden werden. Dann wird zunächst der Dialyatkreislauf befüllt. Nach dem Befüllen des Dialysatkreislaufs erfolgt ein Befüllen des extrakorporalen Blutkreislaufs. Hierfür wechselt die Maschine in einen Bolus-Modus, in welchem durch Betrieb der Blutpumpe Flüssigkeit aus einem Behälter in den extrakorporalen Blutkreislauf gepumpt wird und über einen Entlüfter der venösen Tropfkammer eine Entlüftung erfolgt.

Aus der US 2009/0076433 A1 ist ein ist ein Verfahren zum Befüllen und Spülen eines Blutschlauchsatzes bekannt, für welches die Enden der venösen und der arteriellen Leitung miteinander verbunden werden. Dann werden die venöse und die arterielle Leitung mit Primingflüssigkeit befüllt. Zusätzlich zu der zum Entfernen der Luft aus dem Blutkreislauf eingesetzten Primingflüssigkeit kann auch Dialysat verwendet werden, welches von der Dialysatseite durch die Membran des Filters tritt, um die Entlüftung der Blutseite zu unterstützen.

Wünschenswert wäre es, bei Blutbehandlungsvorrichtungen, die Membranfilter einsetzen, wie dies beispielsweise bei Dialysemaschinen und Dialysatoren der Fall ist, ein automatisches und sicheres Befüllen der Dialysatseite, der Blutseite und des Dialysators zu ermöglichen.

Es ist daher die Aufgabe der vorliegenden Erfindung, eine Blutbehandlungsvorrichtung sowie ein Verfahren der eingangs genannten Art in vorteilhafter Weise weiterzubilden, insbesondere dahingehend, daß der eingesetzte Filter automatisch luftfrei gefüllt werden kann, ohne daß dies weitere Arbeitsschritte durch das Pflegepersonal erfordert.

Diese Aufgabe wird erfindungsgemäß gelöst durch ein Verfahren zum Spülen und/oder zum Befüllen einer Blutbehandlungsvorrichtung mit den Merkmalen des Anspruchs 1. Danach ist vorgesehen, daß bei einem Verfahren zum Spülen und/oder zum Befüllen einer Blutbehandlungsvorrichtung, wobei die Blutbehandlungsvorrichtung wenigstens einen Membranfilter, wenigstens einen ersten Teilkreislauf und wenigstens einen zweiten Teilkreislauf aufweist, wobei der erste und der zweite Teilkreislauf durch den Membranfilter semipermeabel getrennt sind und wobei der erste Teilkreislauf der Dialysekreislauf ist und der zweite Teilkreislauf der extrakorporale Blutkreislauf ist, zumindest derart verfahren wird, daß der zweite Teilkreislauf zur Atmosphäre hin zumindest zeitweise geöffnet ist und wobei Luft über die Membran des Membranfilters von dem ersten in den zweiten Teilkreislauf verdrängt wird. Dabei wird der erste Teilkreislauf zuerst befüllt, während der zweite Teilkreislauf noch leer ist.

Dadurch, daß der zweite Teilkreislauf zur Atmosphäre hin zumindest zeitweise geöffnet ist, denn hierdurch wird insbesondere erreicht, daß im zweiten Teilkreislauf ein nur geringer Gegendruck herrscht, wenn Luft vom ersten Teilkreislauf in den zweiten Teilkreislauf verdrängt werden soll, wie dies während Luft über die Membran des Membranfilters von dem ersten in den zweiten Teilkreislauf verdrängt wird vorgesehen ist. Die Luft kann so einfach über die Membran entfernt werden.

Insbesondere handelt es sich bei dem Spülen und/oder dem Befüllen der Blutbehandlungsvorrichtung um das Priming einer Blutbehandlungsvorrichtung. Der Membranfilter ist vorzugsweise ein Hohlfasermembranfilter. Diese Blutbehandlungsvorrichtung kann vorteilhafterweise eine Dialysemaschine sein. Das für den Befüllvorgang bzw. für das Priming benutzte Fluid kann beispielsweise die Dialyseflüssigkeit sein.

Die Luftentfernung aus dem Membranfilter kann ohne mechanische Manipulation am Membranfilter und ohne technische Änderungen an der Blutbehandlungsvorrichtung erheblich verbessert werden. Insbesondere ist von Vorteil, daß der Membranfilter nunmehr automatisch befüllt und dabei sicher entlüftet werden kann, ohne daß weitere Arbeitsschritte wie ein Umdrehen des Membranfilters z. B. durch das Pflegepersonal erforderlich wären. Es ist möglich, die im ersten Teilkreislauf befindliche Luft über die Membran des Membranfilters während der Befüllungsphase des Membranfilters zu entfernen.

Dies wird insbesondere dadurch begünstigt, daß durch die behutsame Befüllung des ersten Teilkreislaufs und der auf dieser Seite des Teilkreislaufs befindlichen Seite des Membranfilters mit einem Volumenstrom unterhalb eines vorgegebenen Schwellwertes die Membran weiterhin für Luft gut durchlässig bleibt. Denn durch die Verringerung der Benetzung und Verringerung Durchtränkung der Membran des Membranfilters wird erreicht, daß die Luft vergleichsweise ungehindert durch die Membran hindurchtreten kann. Ein derart ungehindertes Übertreten von Luft ist aber nicht mehr ohne weiteres möglich, wenn die Membran des Membranfilters stark mit Fluid benetzt und/oder durchtränkt ist.

Bevorzugt wird daher zumindest zeitweise der erste Teilkreislauf mit einem gleichmäßigen und/oder einem pulsatilen ersten Volumenstrom mit einem Fluid befüllt wird, wobei der Volumenstrom einen vorgegebenen Schwellwert nicht übersteigt, wobei bei diesem Schwellwert noch keine vollständige Benetzung und/oder Durchtränkung der Membran durch das Fluid vorliegt.

Der Membranfilter ist vorzugsweise und insbesondere in Arbeits- bzw. Behandlungsstellung in der bzw. an der Blutbehandlungsvorrichtung im Wesentlichen senkrecht angeordnet. Der erste und der zweite Teilkreislauf sind derart angeordnet, daß die Flußrichtungen durch den Membranfilter gegenläufig sind, d. h. daß der Membranfilter nach dem Gegenstromprinzip durchströmt wird. Dabei strömt das Fluid während des Befüllvorganges im ersten Teilkreislauf von oben nach unten auf der entsprechenden Seite des Membranfilters durch den Membranfilter und im zweiten Teilkreislauf entsprechend umgekehrt, also von unten nach oben auf der entsprechenden Seite des Membranfilters durch den Membranfilter.

Bei dem erfindungsgemäßen Verfahren wird also vorteilhafterweise die Entstehung von Luftblasen im ersten Teilkreislauf reduziert, indem die Entfernung der Luft über die Membran begünstigt wird. Schon während der Füllung des Hohlfasermembranfilters mit dem geringen ersten Volumenstrom, der einen Schwellwert nicht übersteigt, wird Luft über die Membran in den zweiten Teilkreislauf und von dort z. B. in die Umgebung verdrängt.

Weiterhin kann vorgesehen sein, daß während des Befüllens kein Unterdruck am ersten Teilkreislauf angelegt wird.

Der Verzicht auf das Anlegen eines Unterdrucks im ersten Teilkreislauf bewirkt, daß die Hohlfasermembran während des Befüllvorganges länger für Luft durchlässig bleiben kann und auch keine Luft rückwärts aus dem zweiten Teilkreislauf in den ersten Teilkreislauf strömen kann, was zur Folge hätte, daß die Luft nicht mehr entfernt werden könnte.

Dies wird insbesondere dadurch begünstigt, daß alternativ oder vorzugsweise kombiniert durch den ersten Volumenstrom eine langsamere Benetzung der Hohlfasermembranoberfläche bewirkt wird, weiter durch den Verzicht auf das Anlegen eines Unterdrucks im ersten Teilkreislauf ebenfalls eine langsamere Benetzung der Hohlfasermembranoberfläche bewirkt wird und durch das Öffnen des zweiten Teilkreislaufs gegenüber der Atmosphäre erreicht wird, daß sich kein Gegendruck aufbaut, der eine schnellere Benetzung der Hohlfasermembranoberfläche begünstigt. Erfindungsgemäß ist vorgesehen, daß der erste Teilkreislauf der Dialysekreislauf ist und daß der zweite Teilkreislauf der extrakorporale Blutkreislauf ist.

Des weiteren ist denkbar, daß der Membranfilter zunächst auf der Seite des ersten Teilkreislaufs bilanzierend gefüllt wird, wobei bei der bilanzierenden Füllung gleiche Volumina zum und vom Membranfilter gefördert werden und wobei eine Bilanzkammer für die bilanzierende Füllung verwendet wird.

Ferner ist möglich, daß während der Befüllung des Membranfilters auf der Seite des ersten Teilkreislaufs ein oder mehrere Bilanzkammerumschaltungen erfolgen. Besonders vorteilhaft ist es, wenn vorzugsweise drei Bilanzkammerumschaltungen vorgesehen sind.

Darüber hinaus kann vorgesehen sein, daß nach einer vorgegebenen Anzahl von Bilanzkammerumschaltungen Luft über die Membran des Membranfilters von dem ersten in den zweiten Teilkreislauf verdrängt wird. Dadurch kann beispielsweise vermieden werden, daß es zu einem Leerlaufen des Sekundärluftabscheiders kommt, wenn bei der zu befüllenden Blutbehandlungsvorrichtung die Luft aus dem ersten Teilkreislauf zunächst in einen Sekundärluftabscheider gefördert wird.

Des weiteren kann vorgesehen sein, daß der Membranfilter auf der Seite des ersten Teilkreislaufs mit einem ersten Volumenstrom befüllt wird, der ca. 500 ml/min nicht übersteigt. Durch diesen verringerten und gleichmäßigen Volumenstrom des Fluids, mit dem der erste Teilkreislauf befüllt wird und mit dem auch die erste Seite des Membranfilters, die dem ersten Teilkreislauf zugeordnet ist, befüllt wird, wird erreicht, daß die Membran des Membranfilters auf dieser Seite nicht vollständig durchnäßt bzw. durchtränkt wird. Somit kann besonders vorteilhaft sichergestellt werden, daß auch trotz der Befüllung weiterhin ein Luftübertritt über die Membran unproblematisch möglich ist.

Ferner kann vorgesehen sein, daß die Blutbehandlungsvorrichtung einen Sekundärluftabscheider aufweist, in den die Luft aus dem Membranfilter während der Befüllung des Membranfilters auf der Seite des ersten Teilkreislaufs gefördert wird.

Darüber hinaus kann vorgesehen sein, daß Luft aus dem Sekundärluftabscheider und aus dem ersten Teilkreislauf über die semipermeable Membran des Membranfilters in den zweiten Teilkreislauf verdrängt wird.

Des weiteren ist denkbar, daß während Luft über die Membran des Membranfilters von dem ersten in den zweiten Teilkreislauf verdrängt wird in den ersten Teilkreislauf Fluid mit einem Volumenstrom, der größer ist als der erste Volumenstrom, gefördert wird.

Besonders vorteilhaft ist es, wenn mit einem Volumenstrom von ca. 1.300 ml/min gefördert wird. Hierzu kann beispielsweise eine Ladepumpe der Blutbehandlungsvorrichtung eingesetzt werden.

Des weiteren betrifft die vorliegende Erfindung eine Blutbehandlungsvorrichtung mit den Merkmalen des Anspruchs 8. Danach ist vorgesehen, daß die Blutbehandlungsvorrichtung mit wenigstens einem Membranfilter, mit wenigstens einem ersten Teilkreislauf und mit wenigstens einem zweiten Teilkreislauf, mit wenigstens einem ersten Pumpmittel zum Fördern eines Fluids im ersten Teilkreislauf und mit wenigstens einem zweiten Pumpmittel zum Fördern eines Fluids im zweiten Teilkreislauf versehen ist, wobei der erste und der zweite Teilkreislauf durch den Membranfilter semipermeabel getrennt sind, wobei die Blutbehandlungsvorrichtung wenigstens ein Steuerungs- und/oder Regelungsmittel aufweist, so daß die Blutbehandlungsvorrichtung zumindest derart betreibbar ist, daß mittels getrennter Pumpmittel mittelbar und/oder unmittelbar wenigstens der erste und der zweite Teilkreislauf sowie der Membranfilter so spülbar und/oder befüllbar sind, dass der zweite Teilkreislauf zur Atmosphäre hin zumindest zeitweise geöffnet ist und Luft über die Membran des Membranfilters von dem ersten in den zweiten Teilkreislauf verdrängt wird.

Bevorzugt ist die Blutbehandlungsvorrichtung derart ausgebildet, daß während des Befüllens kein Unterdruck am ersten Teilkreislauf angelegt wird.

Weiterhin kann zumindest zeitweise der erste Teilkreislauf mit einem gleichmäßigen und/oder einem pulsatilen Volumenstrom mit einem Fluid befüllbar sein, wobei der Volumenstrom einen vorgegebenen Schwellwert nicht übersteigt, wobei bei diesem Schwellwert noch keine vollständige Benetzung und/oder Durchtränkung der Membran durch das Fluid vorliegt, und/oder wobei

Darüber hinaus kann vorgesehen sein, daß die Blutbehandlungsvorrichtung eine Dialysemaschine ist und/oder daß der Membranfilter ein Hohlfasermembranfilter, insbesondere ein Dialysator ist, und/oder daß der erste Teilkreislauf der Dialysekreislauf ist und/oder daß der zweite Teilkreislauf der extrakorporale Blutkreislauf ist.

Ferner ist möglich, daß das Entlüftungsmittel, mittels dessen der zweite Teilkreislauf zur Umgebung und/oder Atmosphäre hin entlüftbar ist und/oder mittels dessen der zweite Teilkreislauf zur Umgebung und/oder Atmosphäre geöffnet werden kann, ein Klemmenelement und/oder ein Element umfassend eine Hydrophobmembran und/oder ein Ventil ist und/oder umfaßt, wobei vorzugsweise das Ventil ein Entlüftungsventil und/oder ein Ventil ist, daß derart beschaffen ist, daß es die Compliance eines disposablen Schlauchsets der Blutbehandlungsvorrichtung vergrößert, insbesondere ein SingleNeedle-Ventil ist. Das Klemmenelement kann beispielsweise ein Schlauchteil des Disposables sein, der mit einer manuellen Klemme versehen ist, wodurch das Disposable zur Atmosphäre hin geöffnet und geschlossen werden kann.

Außerdem kann vorgesehen sein, daß die Blutbehandlungsvorrichtung wenigstens eine Bilanzkammer aufweist, mittels derer mittelbar und/oder unmittelbar der erste Teilkreislauf bilanzierend befüllbar ist.

Des weiteren kann vorgesehen sein, daß die Blutbehandlungsvorrichtung derart ausgeführt und beschaffen ist, daß der Membranfilter zunächst auf der Seite des ersten Teilkreislaufs bilanzierend gefüllt wird, wobei bei der bilanzierenden Füllung gleiche Volumina zum und vom Membranfilter mittels des ersten Pumpmittels und mittels der Bilanzkammer förderbar sind.

Darüber hinaus ist denkbar, daß der Membranfilter auf der Seite des ersten Teilkreislaufs mittels des ersten Pumpmittels mit einem Volumenstrom befüllt wird, der ca. 500 ml/min nicht übersteigt.

Ferner ist denkbar, daß die Blutbehandlungsvorrichtung einen Sekundärluftabscheider aufweist, in den die Luft aus dem Membranfilter während der Befüllung des Membranfilters auf der Seite des ersten Teilkreislaufs förderbar ist.

Darüber hinaus kann vorgesehen sein, daß mittels der Blutbehandlungsvorrichtung das Verfahren gemäß einem der Ansprüche 1 bis 10 durchführbar ist.

Weitere Einzelheiten und Vorteile der Erfindung sollen nun anhand eines Ausführungsbeispiels näher erläutert werden.

Das erfindungsgemäße Füllverfahren wird vorzugsweise mit einer erfindungsgemäßen Blutbehandlungsmaschine, die eine Dialysemaschine bzw. ein Dialysemonitor ist, durchgeführt, wobei der erste Teilkreislauf der Dialysatkreislauf ist und der zweite Teilkreislauf der extrakorporale Blutkreislauf ist.

Bei der Befüllung bzw. beim Priming des ersten und zweiten Teilkreislaufs sowie des Dialysators wird ausgenutzt, daß durch die behutsame Befüllung des ersten Teilkreislaufs und der auf dieser Seite des Teilkreislaufs befindlichen Seite des Membranfilters mit einem Volumenstrom unterhalb eines vorgegebenen Schwellwertes die Membran weiterhin für Luft gut durchlässig bleibt. Denn durch die Verringerung der Benetzung und Verringerung Durchtränkung der Membran des Membranfilters wird erreicht, daß die Luft vergleichsweise ungehindert durch die Membran hindurchtreten kann. Ein derart ungehindertes Übertreten von Luft ist aber nicht mehr ohne weiteres möglich, wenn die Membran des Membranfilters stark mit Fluid benetzt und/oder durchtränkt ist.

Durch den zusätzlichen Verzicht auf das Anlegen eines Unterdrucks auf der Hydraulikseite wird ebenfalls erreicht, daß eine langsame Benetzung der Hohlfasermembranen des Dialysators erfolgt. Auch wird verhindert, daß die Luft rückwärts aus dem Blutkreislauf auf die Dialysatseite strömt und von dort im folgenden ggf. nicht mehr entfernt werden kann.

Ferner wird ausgenutzt, daß durch den im zweiten Teilkreislauf herrschenden geringen Gegendruck aufgrund der Öffnung des zweiten Teilkreislaufs zur Atmosphäre hin ein Luftübertritt durch die Membran des Dialysators erheblich erleichtert wird.

Im Einzelnen kann folgendermaßen vorgegangen werden:
Der Dialysator wird vor dem Start des Verfahrens zumindest hydraulikseitig konnektiert, wie dies ohnehin bereits Standard ist. Die blutseitige Konnektion ist zur Durchführung des erfindungsgemäßen Verfahrens jedoch nicht notwendig, allerdings ist vorgesehen, daß die Blutseite offen zur Atmosphäre ist. Hierdurch wird insbesondere verhindert, daß sich die dialysatseitige Füllung verschlechtert, da in einem blutseitig verschlossenen Filter durch die auf der Blutseite übertretende Luft ein Druckanstieg stattfindet, welcher einer weiteren Entlüftung der Dialysatseite entgegensteht.

Der extrakorporale Blutkreislauf wird gegen Umgebung geöffnet. Dies kann beispielsweise dadurch erfolgen, daß ein Entlüftungsventil, das im venösen Teil des extrakorporalen Blutkreislaufs z. B. im Bereich oder an einer Tropfkammer bzw. Luftfalle vorgesehen ist, geöffnet wird.

Weiter ist z. B. denkbar und alternativ oder zusätzlich vorgesehen, daß ein Klemmenelement und ein Element umfassend eine Hydrophobmembran als Entlüftungsmittel vorgesehen ist. Das Klemmenelement kann beispielsweise ein Schlauchteil des Disposables sein, der mit einer manuellen Klemme versehen ist, wodurch das Disposable zur Atmosphäre hin geöffnet und geschlossen werden kann.

Auch kann ein Ventil als Entlüftungsventil und/oder ein Ventil vorgesehen sein, daß derart beschaffen ist, daß es die Compliance eines disposablen Schlauchsets der Blutbehandlungsvorrichtung vergrößert, wie z. B. ein SingleNeedle-Ventil. Denkbar ist dann in diesem Zusammenhang insbesondere, das SingleNeedle-Ventil zu öffnen, wenn es sich um eine Blutbehandlungsvorrichtung zur Durchführung eines SingleNeedle-Verfahrens handelt und ein entsprechendes Ventil vorgesehen ist.

Üblicherweise ist der Dialysator während des Befüllens mit dem Schlauchsystem bereits verbunden. Dieses Schlauchsystem ist zudem zur Umgebung nicht offen, da nach dem Einlegen die beiden Schlauchklemmen geschlossen werden und das Ventil zur Entlüftung der venösen Kammer ebenfalls im Grundzustand geschlossen ist.

Beim Füllen, insbesondere von großen Dialysatoren, wird ein Druckanstieg im extrakorporalen Blutkreislauf beobachtet, da Luft von der Dialysatseite auf die Blutseite übertritt. Ermöglicht man das Entweichen der Luft durch das Öffnen des Entlüftungsventils der venösen Kammer, so zeigt sich bereits eine deutlich verbesserte hydraulikseitige Füllung des Filters. Bei einigen Blutbehandlungsvorrichtungen besteht auch die Möglichkeit der Herstellung eines Vakuums im extrakorporalen Blutkreislauf. Dies kann die Entlüftung des Filters unterstützen.

Wird ein Entweichen der Luft aus dem extrakorporalen Blutkreislauf durch das Öffnen z. B. eines Entlüftungsventils ermöglicht, steigt, wie dies beobachtet wurde, das Füllvolumen des Dialysators signifikant an. Damit die Luft über die Membran abgeschieden werden kann, muß die Füllung der Hydraulikseite vor der Füllung der Blutseite erfolgen, da eine Benetzung der Membran (egal von welcher Seite) einen Luftdurchtritt verhindert, und es muß der Druck diesseits der Membran größer sein als jenseits. Ist der Druck auf der zu füllenden Seite des Dialysators geringer, so wird Luft angesaugt und die Füllung wird deutlich verschlechtert.

Der Dialysator wird zunächst hydraulikseitig, also auf der Seite des ersten Teilkreislaufs gefüllt. Die Füllroutine wurde derart modifiziert, daß die ersten fünf Bilanzkammerumschaltungen der Bilanzkammer über den Dialysator mit verringertem und gleichmäßigem Dialysatfluß durchgeführt werden. Dieser beträgt 500 ml/min. Die Luft aus dem Dialysator wird hierbei in den Sekundärluftabscheider gefördert.

Dabei wird berücksichtigt, daß die Entlüftung über die Membran nur dann erfolgen kann, wenn Kapillarteile noch nicht mit Dialysat in Berührung gekommen sind. Sind am Ende der Füllroutine alle Kapillaren naß, kann Luft die Kapillarmembran nicht mehr passieren und die Restluft muß aus dem Dialysator gespült werden. Um zu verhindern, daß schon zu einem frühen Zeitpunkt der Füllroutine alle Kapillaren durchnäßt bzw. durchtränkt sind, muß der Füllvolumenstrom möglichst einen gleichmäßigen Verlauf besitzen.

In dieser Phase arbeitet die Hydraulik bilanzierend, d. h. es werden gleiche Volumina zum und vom Dialysator gefördert. Die Verdrängung von Luft über die Membran spielt somit nur eine untergeordnete Rolle, die vernachlässigbar ist.

Die in den Sekundärluftabscheider gelangte Luft wird dort erkannt und normalerweise durch Anlegen eines Unterdrucks zur Absaugung der Luft abgeschieden. Dieser Vorgang wird auch als Sekundärluftabscheidung bezeichnet. Im hier vorliegenden Fall des Dialysatorfüllens wird jedoch die Sekundärluftabscheidung unterbunden, da das Anlegen eines Unterdrucks im bilanzierenden Kreislauf zu einem unerwünschtem Luftübertritt von der Blut- auf die Dialysatseite führt. Dies kann beispielsweise an abrupten Druckabfällen am venösen Drucksensor jeweils am Ende der Bilanzkammerumschaltungen erkannt werden.

Um ein Leerlaufen des Sekundärluftabscheiders zu vermeiden, erfolgt nach einer gewissen Zahl von Bilanzkammerumschaltungen ein Übergang ins Füllprogramm. Hierbei wird das Bilanziersystem an einem zusätzlichen Ventil geöffnet. Die Bilanzkammer fördert im Füllprogramm zusätzliche Flüssigkeit in den bilanzierenden Kreislauf. Treibende Kraft hierbei ist die Ladepumpe, die einen Fluß von ca. 1.300 ml/min liefert.

Wie am Druckverlauf im extrakorporalen Blutkreislauf erkennbar gemacht werden kann, wird während der ersten Umschaltungen des Füllprogramms Luft in den Blutkreislauf verschoben und kann dort z. B. wegen des Druckabfalls an der Hydrophobmembran im SingleNeedle-Luftport zu einem vorübergehenden Druckanstieg führen.

Nach einigen Umschaltungen ist die Dialysatormembran vollständig benetzt bzw. durchtränkt und es findet kein weiterer Luftübertritt auf die Blutseite mehr statt. Die verbleibende Luft wird durch weitere Füllumschaltungen verdrängt, bis wieder Flüssigkeit erkannt werden kann.

Nach insgesamt 15 Umschaltungen endet die Dialysator-Füllung. Grundsätzlich kann bei kleineren Filtern die Befüllung natürlich auch früher beendet werden. Ab der sechsten Bilanzkammerumschaltung wird während der Normalschaltungen mit dem vom Gerät verfügbaren Maximalfluß gefüllt (1000ml/min), denn durch den hohen Spitzenfluß während der Füllumschaltungen sind die Kapillaren dann ohnehin großflächig benetzt.

Weitere nutzbare Effekte dieses vorbeschriebenen Verfahren können noch darin bestehen, daß eine Auswertung der Bilanzkammerumschaltungen im Füllprogramm benutzt wird, um Aussagen zum dialysatseitigen Füllvolumen des Filters zu liefern. Zusammen mit der Information des blutseitigen Füllvolumens (kann beim Füllen der Blutseite ermittelt werden) lässt sich gegebenenfalls maschinenseitig der Dialysator bestimmen. Ferner ermöglicht eine Auswertung der venösen (und Prefilter-) Druckpulse zu erkennen, ob die Blutschläuche am Dialysator angeschlossen wurden, ohne daß Flüssigkeit austritt.

## Patentansprüche

1. Verfahren zum Spülen und/oder zum Befüllen einer Blutbehandlungsvorrichtung, insbesondere für das Priming einer Blutbehandlungsvorrichtung, wobei die Blutbehandlungsvorrichtung wenigstens einen Membranfilter, insbesondere einen Hohlfasermembranfilter, wenigstens einen ersten Teilkreislauf und wenigstens einen zweiten Teilkreislauf aufweist, wobei der erste und der zweite Teilkreislauf durch den Membranfilter semipermeabel getrennt sind, wobei der erste Teilkreislauf der Dialysekreislauf ist und der zweite Teilkreislauf der extrakorporale Blutkreislauf ist,
wobei der erste Teilkreislauf zuerst befüllt wird, während der zweite Teilkreislauf noch leer ist,
**dadurch gekennzeichnet,**
**dass** der zweite Teilkreislauf zur Atmosphäre hin zumindest zeitweise geöffnet ist oder in diesem ein Vakuum hergestellt wird und wobei Luft über die Membran des Membranfilters von dem ersten in den zweiten Teilkreislauf verdrängt wird.

2. Verfahren nach Anspruch 1, wobei
zumindest zeitweise der erste Teilkreislauf mit einem gleichmäßigen und/oder einem pulsatilen ersten Volumenstrom mit einem Fluid befüllt wird, wobei der Volumenstrom einen vorgegebenen Schwellwert nicht übersteigt, wobei bei diesem Schwellwert noch keine vollständige Benetzung und/oder Durchtränkung der Membran durch das Fluid vorliegt
und/oder wobei während des Befüllens kein Unterdruck am ersten Teilkreislauf angelegt wird.

3. Verfahren nach Anspruch 1 oder 2, **dadurch gekennzeichnet, daß** der Membranfilter zunächst auf der Seite des ersten Teilkreislaufs bilanzierend gefüllt wird, wobei bei der bilanzierenden Füllung gleiche Volumina zum und vom Membranfilter gefördert werden und wobei eine Bilanzkammer für die bilanzierende Füllung verwendet wird, wobei bevorzugt während der Befüllung des Membranfilters auf der Seite des ersten Teilkreislaufs ein oder mehrere Bilanzkammerumschaltungen erfolgen, wobei vorzugsweise drei Bilanzkammerumschaltungen vorgesehen sind, wobei weiterhin bevorzugt nach einer vorgegebenen Anzahl von Bilanzkammerumschaltungen Luft über die Membran des Membranfilters von dem ersten in den zweiten Teilkreislauf verdrängt wird.

4. Verfahren nach einem der vorhergehenden Ansprüche, **dadurch gekennzeichnet, daß** der Membranfilter auf der Seite des ersten Teilkreislaufs mit einem ersten Volumenstrom teilweise befüllt wird, der ca. 500 ml/min nicht übersteigt.

5. Verfahren nach einem der vorhergehenden Ansprüche, **dadurch gekennzeichnet, daß** die Blutbehandlungsvorrichtung einen Sekundärluftabscheider aufweist, in den die Luft aus dem Membranfilter während der Befüllung des Membranfilters auf der Seite des ersten Teilkreislaufs gefördert wird.

6. Verfahren nach einem der vorhergehenden Ansprüche, **dadurch gekennzeichnet, daß** während Luft über die Membran des Membranfilters von dem ersten in den zweiten Teilkreislauf verdrängt wird in den ersten Teilkreislauf Fluid mit einem Volumenstrom, der größer ist als der erste Volumenstrom, gefördert wird.

7. Verfahren nach Anspruch 6, **dadurch gekennzeichnet, daß** mit einem Volumenstrom von ca. 1.300 ml/min gefördert wird.

8. Blutbehandlungsvorrichtung mit wenigstens einem Membranfilter, mit wenigstens einem ersten Teilkreislauf und mit wenigstens einem zweiten Teilkreislauf, mit wenigstens einem ersten Pumpmittel zum Fördern eines Fluids im ersten Teilkreislauf und mit wenigstens einem zweiten Pumpmittel zum Fördern eines Fluids im zweiten Teilkreislauf, wobei der erste und der zweite Teilkreislauf durch den Membranfilter semipermeabel getrennt sind, wobei der erste Teilkreislauf der Dialysekreislauf ist und der zweite Teilkreislauf der extrakorporale Blutkreislauf ist,
wobei die Blutbehandlungsvorrichtung wenigstens ein Steuerungs- und/oder Regelungsmittel aufweist, so daß die Blutbehandlungsvorrichtung zumindest derart betreibbar ist, daß mittels getrennter Pumpmittel mittelbar und/oder unmittelbar wenigstens der erste und der zweite Teilkreislauf sowie der Membranfilter so spülbar und/oder befüllbar sind,
- dass der erste Teilkreislauf zuerst befüllt wird, während der zweite Teilkreislauf noch leer ist,
- dass der zweite Teilkreislauf zur Atmosphäre hin zumindest zeitweise geöffnet ist oder in diesem ein Vakuum hergestellt wird und
- dass Luft über die Membran des Membranfilters von dem ersten in den zweiten Teilkreislauf verdrängt wird.

9. Blutbehandlungsvorrichtung nach Anspruch 8, **dadurch gekennzeichnet, daß** die Blutbehandlungsvorrichtung eine Dialysemaschine ist und/oder daß der Membranfilter ein Hohlfasermembranfilter, insbesondere ein Dialysator ist, und/oder daß zumindest zeitweise der erste Teilkreislauf mit einem gleichmäßigen und/oder einem pulsatilen Volumenstrom mit einem Fluid befüllbar ist, wobei der Volumenstrom einen vorgegebenen Schwellwert nicht übersteigt, wobei bei diesem Schwellwert noch keine vollständige Benetzung und/oder Durchtränkung der Membran durch das Fluid vorliegt, und/oder dass die Blutbehandlungsvorrichtung derart ausgebildet ist, daß während des Befüllens kein Unterdruck am ersten Teilkreislauf angelegt wird.

10. Blutbehandlungsvorrichtung nach Anspruch 8 oder 9, **dadurch gekennzeichnet, daß** das Entlüftungsmittel, mittels dessen der zweite Teilkreislauf zur Umgebung und/oder Atmosphäre hin entlüftbar ist und/oder mittels dessen der zweite Teilkreislauf zur Umgebung und/oder Atmosphäre geöffnet werden kann, ein Klemmenelement und/oder ein Element umfassend eine Hydrophobmembran und/oder ein Ventil ist und/oder umfaßt, wobei vorzugsweise das Ventil ein Entlüftungsventil und/oder ein Ventil ist, daß derart beschaffen ist, daß es die Compliance eines disposablen Schlauchsets der Blutbehandlungsvorrichtung vergrößert, insbesondere ein SingleNeedle-Ventil ist.

11. Blutbehandlungsvorrichtung nach einem der Ansprüche 8 bis 10, **dadurch gekennzeichnet, daß** die Blutbehandlungsvorrichtung wenigstens eine Bilanzkammer aufweist, mittels derer mittelbar und/oder unmittelbar der erste Teilkreislauf bilanzierend befüllbar ist.

12. Blutbehandlungsvorrichtung nach Anspruch 11, **dadurch gekennzeichnet, daß** die Blutbehandlungsvorrichtung derart ausgeführt und beschaffen ist, daß der Membranfilter zunächst auf der Seite des ersten Teilkreislaufs bilanzierend gefüllt wird, wobei bei der bilanzierenden Füllung gleiche Volumina zum und vom Membranfilter mittels des ersten Pumpmittels und mittels der Bilanzkammer förderbar sind.

13. Blutbehandlungsvorrichtung nach einem der Ansprüche 8 bis 12, **dadurch gekennzeichnet, daß** der Membranfilter auf der Seite des ersten Teilkreislaufs mittels des ersten Pumpmittels mit einem Volumenstrom befüllt wird, der ca. 500 ml/min nicht übersteigt.

14. Blutbehandlungsvorrichtung nach einem der Ansprüche 8 bis 13, **dadurch gekennzeichnet, daß** die Blutbehandlungsvorrichtung einen Sekundärluftabscheider aufweist, in den die Luft aus dem Membranfilter während der Befüllung des Membranfilters auf der Seite des ersten Teilkreislaufs förderbar ist.

15. Blutbehandlungsvorrichtung nach einem der Ansprüche 8 bis 14, **dadurch gekennzeichnet, daß** mittels der Blutbehandlungsvorrichtung das Verfahren gemäß einem der Ansprüche 1 bis 7 durchführbar ist.

## Claims

1. A method for rinsing and/or for filling a blood treatment device, in particular for priming a blood treatment device, wherein the blood treatment device includes at least one membrane filter, in particular a hollow fiber membrane filter, at least one first partial circuit and at least one second partial circuit, wherein the first and the second partial circuit are separated by the membrane filter in a semipermeable manner, wherein the first partial circuit is the dialysis circuit and the second partial circuit is the extracorporeal blood circuit,
wherein the first partial circuit is first filled, while the second partial circuit is still empty,
**characterized in**
**that** the second partial circuit is at least temporarily open towards the atmosphere or a vacuum is produced in the same, and wherein air is displaced from the first and into the second partial circuit via the membrane of the membrane filter.

2. The method according to claim 1, wherein
at least temporarily the first partial circuit is filled with a fluid with a uniform and/or a pulsatile first volumetric flow rate, wherein the volumetric flow rate does not exceed a specified threshold value, wherein at this threshold value the fluid has not yet completely wetted and/or soaked the membrane, and/or wherein during filling no negative pressure is applied to the first partial circuit.

3. The method according to claim 1 or 2, **characterized in that** the membrane filter first is filled in a balancing manner on the side of the first partial circuit, wherein during the balancing filling equal volumes are delivered to and from the membrane filter and wherein a balancing chamber is used for the balancing filling, wherein preferably while filling the membrane filter on the side of the first partial circuit one or more balancing chamber switching operations are effected, wherein preferably three balancing chamber switching operations are provided, wherein furthermore preferably after a specified number of balancing chamber switching operations air is displaced from the first into the second partial circuit via the membrane of the membrane filter.

4. The method according to any of the preceding claims, **characterized in that** on the side of the first partial circuit the membrane filter is partly filled with a first volumetric flow rate which does not exceed about 500 ml/min.

5. The method according to any of the preceding claims, **characterized in that** the blood treatment device includes a secondary air separator into which the air from the membrane filter is delivered while filling the membrane filter on the side of the first partial circuit.

6. The method according to any of the preceding claims, **characterized in that** while air is displaced from the first into the second partial circuit via the membrane of the membrane filter, fluid is delivered into the first partial circuit with a volumetric flow rate which is greater than the first volumetric flow rate.

7. The method according to claim 6, **characterized in that** delivery is effected with a volumetric flow rate of about 1300 ml/min.

8. A blood treatment device with at least one membrane filter, with at least one first partial circuit and with at least one second partial circuit, with at least one first pumping means for delivering a fluid in the first partial circuit and with at least one second pumping means for delivering a fluid in the second partial circuit, wherein the first and the second partial circuit are separated by the membrane filter in a semipermeable manner, wherein the first partial circuit is the dialysis circuit and the second partial circuit is the extracorporeal blood circuit,
wherein the blood treatment device includes at least one control and/or regulating means, so that the blood treatment device at least is operable such that by means of separate pumping means at least the first and the second partial circuit as well as the membrane filter can directly and/or indirectly be rinsed and/or filled such,
- that the first partial circuit is first filled, while the second partial circuit is still empty,
- that the second partial circuit is at least temporarily open towards the atmosphere or a vacuum is produced in the same, and
- that air is displaced from the first into the second partial circuit via the membrane of the membrane filter.

9. The blood treatment device according to claim 8, **characterized in that** the blood treatment device is a dialysis machine and/or that the membrane filter is a hollow fiber membrane filter, in particular a dialyzer, and/or that at least temporarily the first partial circuit can be filled with a fluid with a uniform and/or a pulsatile volumetric flow rate, wherein the volumetric flow rate does not exceed a specified threshold value, wherein at this threshold value the fluid has not yet completely wetted and/or soaked the membrane, and/or that the blood treatment device is configured such that during filling no negative pressure is applied to the first partial circuit.

10. The blood treatment device according to claim 8 or 9, **characterized in that** the venting means by means of which the second partial circuit can be vented towards the surroundings and/or the atmosphere and/or by means of which the second partial circuit can be opened towards the surroundings and/or the atmosphere is and/or comprises a clamp element and/or an element comprising a hydrophobic membrane and/or a valve, wherein preferably the valve is a vent valve and/or a valve which is designed such that it increases the compliance of a disposable tubing set of the blood treatment device, in particular is a single-needle valve.

11. The blood treatment device according to any of claims 8 to 10, **characterized in that** the blood treatment device includes at least one balancing chamber by means of which the first partial circuit can directly and/or indirectly be filled in a balancing manner.

12. The blood treatment device according to claim 11, **characterized in that** the blood treatment device is configured and designed such that the membrane filter first is filled in a balancing manner on the side of the first partial circuit, wherein during the balancing filling equal volumes can be delivered to and from the membrane filter by means of the first pumping means and by means of the balancing chamber.

13. The blood treatment device according to any of claims 8 to 12, **characterized in that** on the side of the first partial circuit the membrane filter is filled by means of the first pumping means with a volumetric flow rate which does not exceed about 500 ml/min.

14. The blood treatment device according to any of claims 8 to 13, **characterized in that** the blood treatment device includes a secondary air separator into which the air from the membrane filter can be delivered while filling the membrane filter on the side of the first partial circuit.

15. The blood treatment device according to any of claims 8 to 14, **characterized in that** by means of the blood treatment device the method according to any of claims 1 to 7 can be performed.

## Revendications

1. Procédé de rinçage et/ou de remplissage d'un dispositif de traitement du sang, en particulier pour amorcer un dispositif de traitement du sang, dans lequel le dispositif de traitement du sang comprend au moins un filtre à membrane, en particulier un filtre à membrane à fibres creuses, au moins un premier circuit partiel et au moins un deuxième circuit partiel, le premier et le deuxième circuit partiel étant séparés de manière semi-perméable par le filtre à membrane, le premier circuit partiel étant un circuit de dialyse et le deuxième circuit partiel étant le circuit de sang extracorporel,
dans lequel le premier circuit partiel est rempli d'abord, pendant que le deuxième circuit partiel est encore vide,
**caractérisé en ce que**
le deuxième circuit partiel est ouvert vers l'atmosphère au moins temporairement ou un vide est produit dans celui-ci, et dans lequel de l'air est déplacé du premier dans le deuxième circuit partiel à travers la membrane du filtre à membrane.

2. Procédé selon la revendication 1, dans lequel
au moins temporairement le premier circuit partiel est rempli d'un fluide avec un premier débit volumétrique uniforme et/ou pulsatile, le débit volumétrique ne dépassant pas une valeur seuil spécifiée, à cette valeur seuil le fluide n'ayant pas complètement mouillé et/ou imprégné la membrane, et/ou dans lequel pendant le remplissage pas de pression négative est appliquée au premier circuit partiel.

3. Procédé selon la revendication 1 ou 2, **caractérisé en ce que** le filtre à membrane est rempli d'abord sur le côté du premier circuit partiel d'une manière équilibrante, des volumes égaux étant refoulés vers et depuis le filtre à membrane pendant le remplissage équilibrant et une chambre d'équilibrage étant utilisée pour le remplissage équilibrant, dans lequel pendant le remplissage du filtre à membrane une ou plusieurs opérations de commutation de la chambre d'équilibrage sont effectuées de préférence sur le côté du premier circuit partiel, de préférence trois opérations de commutation de la chambre d'équilibrage étant prévues, dans lequel en outre de préférence de l'air est déplacé du premier dans le deuxième circuit partiel à travers la membrane du filtre à membrane après un nombre spécifié d'opérations de commutation de la chambre d'équilibrage.

4. Procédé selon l'une quelconque des revendications précédentes, **caractérisé en ce que** le filtre à membrane sur le côté du premier circuit partiel est rempli partiellement avec un premier débit volumétrique, qui ne dépasse pas environ 500 ml/min.

5. Procédé selon l'une quelconque des revendications précédentes, **caractérisé en ce que** le dispositif de traitement du sang comprend un séparateur d'air secondaire, dans lequel l'air du filtre à membrane est refoulé pendant le remplissage du filtre à membrane sur le côté du premier circuit partiel.

6. Procédé selon l'une quelconque des revendications précédentes, **caractérisé en ce que** pendant de l'air est déplacé du premier dans le deuxième circuit partiel à travers la membrane du filtre à membrane du fluide est refoulé dans le premier circuit partiel avec un débit volumétrique supérieur au premier débit volumétrique.

7. Procédé selon la revendication 6, **caractérisé en ce que** le refoulement est effectué avec un débit volumétrique d'environ 1300 ml/min.

8. Dispositif de traitement du sang avec au moins un filtre à membrane, avec au moins un premier circuit partiel et avec au moins un deuxième circuit partiel, avec au moins un premier moyen de pompage pour refouler un fluide dans le premier circuit partiel et avec au moins un deuxième moyen de pompage pour refouler un fluide dans le deuxième circuit partiel, le premier et le deuxième circuit partiel étant séparés de manière semi-perméable par le filtre à membrane, le premier circuit partiel étant le circuit de dialyse et le deuxième circuit partiel étant le circuit de sang extracorporel,
dans lequel le dispositif de traitement du sang comprend au moins un moyen de commande et/ou de régulation, de manière que le dispositif de traitement du sang est actionnable au moins de telle sorte qu'au moyen des moyens de pompage séparés au moins le premier et le deuxième circuit partiel ainsi que le filtre à membrane peut être rincé et/ou rempli directement et/ou indirectement, de telle sorte
- que le premier circuit partiel est d'abord rempli, pendant que le deuxième circuit partiel est encore vide,
- que le deuxième circuit partiel est ouvert vers l'atmosphère au moins temporairement ou un vide est produit dans celui-ci, et
- que de l'air est déplacé du premier dans le deuxième circuit partiel à travers la membrane du filtre à membrane.

9. Dispositif de traitement du sang selon la revendication 8, **caractérisé en ce que** le dispositif de traitement du sang est une machine de dialyse et/ou que le filtre à membrane est un filtre à membrane à fibres creuses, en particulier un dialyseur, et/ou qu'au moins temporairement le premier circuit partiel peut être rempli d'un fluide avec un débit volumétrique uniforme et/ou pulsatile, le débit volumétrique ne dépassant pas une valeur seuil spécifiée, le fluide n'ayant pas complètement mouillé et/ou imprégné la membrane à cette valeur seuil, et/ou que le dispositif de traitement du sang est configuré de telle sorte que pendant le remplissage pas de pression négative est appliquée au premier circuit partiel.

10. Dispositif de traitement du sang selon la revendication 8 ou 9, **caractérisé en ce que** le moyen de désaération, au moyen duquel le deuxième circuit partiel peut être évacué vers l'environnement et/ou l'atmosphère et/ou au moyen duquel le deuxième circuit partiel peut être ouvert vers l'environnement et/ou l'atmosphère, est et/ou comporte un élément de serrage et/ou un élément comportant une membrane hydrophobe et/ou une soupape, la soupape de préférence étant une soupape de purge et/ou une soupape qui est conçue de telle sorte qu'elle augmente la compliance d'un ensemble de tubulures à usage unique du dispositif de traitement du sang, en particulier une soupape monoaiguille.

11. Dispositif de traitement du sang selon l'une quelconque des revendications 8 à 10, **caractérisé en ce que** le dispositif de traitement du sang comprend au moins une chambre d'équilibrage, au moyen de laquelle le premier circuit partiel peut être rempli d'une manière équilibrante directement et/ou indirectement.

12. Dispositif de traitement du sang selon la revendication 11, **caractérisé en ce que** le dispositif de traitement du sang est configuré et conçu de telle sorte que le filtre à membrane est d'abord rempli d'une manière équilibrante sur le côté du premier circuit partiel, des volumes égaux pouvant être refoulés vers et depuis le filtre à membrane au moyen du premier moyen de pompage et au moyen de la chambre d'équilibrage pendant le remplissage équilibrant.

13. Dispositif de traitement du sang selon l'une quelconque des revendications 8 à 12, **caractérisé en ce que** sur le côté du premier circuit partiel le filtre à membrane est rempli au moyen du premier moyen de pompage avec un débit volumétrique, qui ne dépasse pas environ 500 ml/min.

14. Dispositif de traitement du sang selon l'une quelconque des revendications 8 à 13, **caractérisé en ce que** le dispositif de traitement du sang comprend un séparateur d'air secondaire, dans lequel l'air du filtre à membrane peut être refoulé pendant le remplissage du filtre à membrane sur le côté du premier circuit partiel.

15. Dispositif de traitement du sang selon l'une quelconque des revendications 8 à 14, **caractérisé en ce qu'**au moyen du dispositif de traitement du sang le procédé selon l'une quelconque des revendications 1 à 7 peut être réalisé.
